# EUROPEAN PATENT APPLICATION

(11) **EP 3 816 279 A1**
(43) Date of publication of application: **05.05.2021**
(21) Application number: 19825687.7
(22) Date of filing: 27.06.2019
(51) Int. Cl.: C12N 5/10, C12N 5/0735

(54) **ADDITIVE FOR CULTURING STEM CELLS, CULTURING MEDIUM, AND CULTURING METHOD**

(30) Priority: 27.06.2018 JP 2018122532
(71) Applicant: AJINOMOTO CO., INC., Chuo-ku Tokyo 104-8315 (JP)
(72) Inventor: ITO, Kenichiro, Kawasaki-shi, Kanagawa 210-8681 (JP)
(74) Representative: Hoffmann Eitle
(86) International application number: PCT/JP2019/025666
(87) International publication number: WO 2020/004571

(57) **Abstract**

The present invention is characterized by an additive for culturing stem cells containing a polysaccharide other than dextran sulfate having a molecular weight of 4,000 kDa to 40,000 kDa, a medium for culturing stem cells, containing a polysaccharide other than dextran sulfate having a molecular weight of 4,000 kDa to 40,000 kDa, and a method for culturing a stem cell, including suspension culturing the stem cell in a medium for culturing stem cells containing a polysaccharide other than dextran sulfate having a molecular weight of 4,000 kDa to 40,000 kDa.

According to the present invention, in suspension culture of stem cells, the forming rate of cell aggregates of the stem cells can be improved, the shape thereof can be controlled, and also the proliferation rate and the rate of maintaining an undifferentiated state of the stem cells can be improved.

## Description

### [Technical Field]

The present invention relates to additives for culturing stem cells that are added to a medium and the like to culture stem cells, media for culturing stem cells, and methods for culturing stem cells.

### [Background Art]

Human stem cells, including embryonic stem cells and induced pluripotent stem cells, have been proliferated and maintained by adhesion culture using human-type recombinant matrix such as matrigel, vitronectin and laminin as scaffold materials.

However, to apply stem cells to research, production, medical treatment, and the like, a culture method for efficiently proliferating them is required. As a method for culturing a large amount of stem cells, a method of suspension culture in the state of a cell aggregate is widely used instead of the above-mentioned adhesion culture.

A suspension culture device with an improved stirring bar, and a culture device in which a culture medium flows by driving the culture vessel itself have been developed so that, during suspension culture of stem cells, cell death will not be caused by shear stress due to the flow of the culture medium, while suppressing excessive cell aggregation.

In the culture of cell aggregates, it is desirable to control the forming rate and the shape of cell aggregates to ensure cell quality and construct an optimal process. In the case of stem cells, maintenance of undifferentiated potential free from differentiation by culture is required.

As such a control method, control using a medium, a medium component, or a culture vessel is considered, and culture substrates and culture media have been developed assuming scale-up to mass culture. For example, culture substrates free of components derived from heterozoice animals such as laminin fragment (LM-E8), vitronectin fragment (VTN-N) and the like (xeno-free), and xeno-free, and further albumin-free, serum-free media have been developed (non-patent documents 1 and 2).

However, a control method that is particularly applicable to mass suspension culture and does not deteriorate the quality of cells is desirable.

Recently, it has been reported that dextran sulfate has the effect of controlling the aggregation of pluripotent stem cells (human embryonic stem cells) and promotes the formation of uniform and small cell aggregates (non-patent document 3).

However, while non-patent document 3 describes the above-mentioned effect of high-molecular-weight dextran sulfate of 4,000 kDa to 40,000 kDa, it does not specifically refer to the effect of low-molecular-weight dextran sulfate and polysaccharides other than dextran sulfate.

### [Document List]

### [non-patent documents]

non-patent document 1: seibutsu-kogaku kaishi 92 (9) 469-472 (2014)
non-patent document 2: seibutsu-kogaku kaishi 92 (9) 487-490 (2014)
non-patent document 3: Biotechnology and Bioengineering 2018, 1-6

### [Summary of Invention]

### [Technical Problem]

The present invention has been made under the above-mentioned situation.

That is, the present invention aims to provide additives for culturing, that are preferable for, in suspension culture of stem cells, improving the forming rate of cell aggregates of stem cells, controlling the shape thereof, and further improving the proliferation rate and the rate of maintaining an undifferentiated state of stem cells; media for culturing stem cells; and methods for culturing stem cells.

### [Solution to Problem]

The present inventors have conducted intensive studies in an attempt to solve the above-mentioned problems and found that the proliferation rate and survival rate of stem cells can be improved, the forming rate of cell aggregates can be improved, the shape of the cell aggregate can be favorably controlled, and the rate of maintaining an undifferentiated state can be improved by adding an additive containing a polysaccharide other than high-molecular-weight dextran sulfate such as sulfated polysaccharides (e.g., low-molecular-weight dextran sulfate and the like) to a medium for culturing stem cells, and performing suspension culture of the stem cells, or performing suspension culture of stem cells in a medium for culturing stem cells containing a polysaccharide other than high-molecular-weight dextran sulfate such as sulfated polysaccharides (e.g., low-molecular-weight dextran sulfate and the like), and completed the present invention.

That is, the present invention relates to the following.
[1] An additive for culturing stem cells comprising a polysaccharide other than dextran sulfate having a molecular weight of 4,000 kDa to 40,000 kDa.
[2] The additive of [1], wherein the stem cell is one kind or two or more kinds selected from the group consisting of an adult stem cell, an embryonic stem cell and an induced pluripotent stem cell.
[3] The additive of [1] or [2], wherein the polysaccharide other than the dextran sulfate having a molecular weight of 4,000 kDa to 40,000 kDa is the aforementioned polysaccharide other than the dextran sulfate, and is an anionic polysaccharide having a negatively-charged functional group or a salt thereof.
[4] The additive of any of [1] to [3], wherein the polysaccharide other than the dextran sulfate having a molecular weight of 4,000 kDa to 40,000 kDa is a sulfated polysaccharide other than the aforementioned dextran sulfate, or a salt thereof.
[5] The additive of any of [1] to [4], wherein the polysaccharide other than the dextran sulfate having a molecular weight of 4,000 kDa to 40,000 kDa is one kind or two or more kinds selected from the group consisting of heparin and a salt thereof, and dextran sulfate having a weight average molecular weight of 5,000 to 50,000 and a salt thereof.
[6] The additive of any of [1] to [5], wherein the additive is added to a medium for culturing stem cells.
[7] The additive of [6], wherein the additive is added such that a concentration of the polysaccharide other than the dextran sulfate having a molecular weight of 4,000 kDa to 40,000 kDa is 1 µg/mL to 1 mg/mL relative to the total amount of the medium.
[8] A medium for culturing stem cells, comprising a polysaccharide other than dextran sulfate having a molecular weight of 4,000 kDa to 40,000 kDa.
[9] The medium of [8], wherein the medium is for culturing one kind or two or more kinds selected from the group consisting of an adult stem cell, an embryonic stem cell and an induced pluripotent stem cell.
[10] The medium of [8] or [9], wherein the polysaccharide other than the dextran sulfate having a molecular weight of 4,000 kDa to 40,000 kDa is the aforementioned polysaccharide other than the dextran sulfate, and is an anionic polysaccharide having a negatively-charged functional group or a salt thereof.
[11] The medium of any of [8] to [10], wherein the polysaccharide other than the dextran sulfate having a molecular weight of 4,000 kDa to 40,000 kDa is a sulfated polysaccharide other than the aforementioned dextran sulfate, or a salt thereof.
[12] The medium of any of [8] to [11], wherein the polysaccharide other than the dextran sulfate having a molecular weight of 4,000 kDa to 40,000 kDa is one kind or two or more kinds selected from the group consisting of heparin and a salt thereof, and dextran sulfate having a weight average molecular weight of 5,000 to 50,000 and a salt thereof.
[13] The medium of any of [8] to [12], wherein a content of the polysaccharide other than dextran sulfate having a molecular weight of 4,000 kDa to 40,000 kDa is 1 µg/mL to 1 mg/mL.
[14] The medium of any of [8] to [13], wherein the medium is a feeder-free medium.
[15] The medium of [14], wherein the medium is a serum-free medium.
[16] A method for culturing a stem cell, comprising suspension culturing the stem cell in a medium for culturing stem cells comprising a polysaccharide other than dextran sulfate having a molecular weight of 4,000 kDa to 40,000 kDa.
[17] The method of [16], wherein the stem cell is one kind or two or more kinds selected from the group consisting of an adult stem cell, an embryonic stem cell and an induced pluripotent stem cell.
[18] The method of [16] or [17], wherein the polysaccharide other than the dextran sulfate having a molecular weight of 4,000 kDa to 40,000 kDa is the aforementioned polysaccharide other than the dextran sulfate, and is an anionic polysaccharide having a negatively-charged functional group or a salt thereof.
[19] The method of any of [16] to [18], wherein the polysaccharide other than the dextran sulfate having a molecular weight of 4,000 kDa to 40,000 kDa is a sulfated polysaccharide other than the aforementioned dextran sulfate, or a salt thereof.
[20] The method of any of [16] to [19], wherein the polysaccharide other than the dextran sulfate having a molecular weight of 4,000 kDa to 40,000 kDa is one kind or two or more kinds selected from the group consisting of heparin and a salt thereof, and dextran sulfate having a weight average molecular weight of 5,000 to 50,000 and a salt thereof.
[21] The method of any of [16] to [20], wherein a content of the polysaccharide other than dextran sulfate having a molecular weight of 4,000 kDa to 40,000 kDa in the medium for culturing stem cells is 1 µg/mL to 1 mg/mL.
[22] The method of any of [16] to [21], wherein the medium for culturing stem cells is a feeder free medium.
[23] The method of [22], wherein the medium for culturing stem cells is a serum-free medium.

### [Advantageous Effects of Invention]

According to the present invention, an additive and a medium for culturing that are preferable for suspension culture of stem cells, and a method for culturing stem cells can be provided.

Therefore, the present invention can improve the proliferation rate and the survival rate of stem cells in suspension culture of the stem cells, improve the forming rate of cell aggregates with controlled size and shape, and further improve the rate of maintaining an undifferentiated state.

### [Brief Description of Drawings]

Fig. 1 shows the effects of sodium heparin and sodium dextran sulfate (average molecular weight=5,000) on cell aggregate formation and cell condition in the stirring culture of human iPS cells in Example 1. In the Figure, "Dextran sulfate" denotes sodium dextran sulfate.
Fig. 2 shows the effects of heparin sodium when passage was repeated in the stirring passage culture of human iPS cells in Example 2.
Fig. 3 shows the effects of sodium dextran sulfate (average molecular weight=5,000) when passage was repeated in the stirring passage culture of human iPS cells in Example 3. In the Figure, "Dextran sulfate" denotes sodium dextran sulfate.
Fig. 4 shows the effects of various heparins in the stirring culture of human iPS cells in Example 4.
Fig. 5 shows the effects of sodium dextran sulfate with a different molecular weight in the stirring culture of human iPS cells in Example 5. In the Figure, "Dextran sulfate" denotes sodium dextran sulfate.
Fig. 6 shows the effects of heparin sodium in the stirring culture of human iPS cells in various stem cell culture media in Example 6.
Fig. 7 shows the effects of sodium dextran sulfate (average molecular weight=5,000) in the stirring culture of human iPS cells in various stem cell culture media in Example 7. In the Figure, "Dextran sulfate" denotes sodium dextran sulfate.
Fig. 8 shows the heparin sodium concentration dependence of the cell aggregate forming rate-improving effect in the stirring culture of human iPS cells in Example 8.
Fig. 9 shows the sodium dextran sulfate (average molecular weight=5,000) concentration dependence of the cell aggregate forming rate-improving effect in the stirring culture of human iPS cells in Example 9. In the Figure, "Dextran sulfate" denotes sodium dextran sulfate.
Fig. 10 shows the effects of heparin sodium on cell aggregate formation and cell condition in the shaking culture of human iPS cells in Example 10.
Fig. 11 shows the sodium dextran sulfate (average molecular weight=5,000) concentration dependence of the cell aggregate forming rate-improving effect in the stirring culture of human iPS cells in Example 11.

### [Description of Embodiments]

The present invention provides an additive for culturing stem cells that can be added to a medium for culturing stem cells (hereinafter to be also referred to as "the additive of the present invention" in the present specification).

As used herein, the "stem cell" refers to a cell that has self-renewal ability and the ability to differentiate into another type of cell and can proliferate endlessly.

Examples include adult stem cell such as hematopoietic stem cell, satellite cell, neural stem cell, mesenchymal stem cell, mammary gland stem cell, olfactory mucosa stem cell, neural crest stem cell, liver stem cell, pancreatic stem cell, muscle stem cell, germ stem cell, the intestine stem cell, hair follicle stem cell and the like; pluripotent stem cell such as embryonic stem cell (ES cell), embryonic tumor cell, embryonic germ cell, induced pluripotent stem cell (iPS cell) and the like; cancer stem cell and the like.

The additive of the present invention is preferably used for culturing adult stem cells, embryonic stem cells and induced pluripotent stem cells, and more preferably used for culturing embryonic stem cells and induced pluripotent stem cells.

The additive of the present invention contains a polysaccharide other than dextran sulfate having a molecular weight of 4,000 kDa to 40,000 kDa.

In the present invention, the "polysaccharide other than dextran sulfate having a molecular weight of 4,000 kDa to 40,000 kDa" refers to a substance in which multiple (two or more) monosaccharide molecules are bound by a glycoside bond, and which is other than high-molecular-weight dextran sulfate having a molecular weight of 4,000 kDa to 40,000 kDa. In the following, it is sometimes indicated as "the polysaccharide in the present invention" in the present specification.

Examples of the monosaccharide constituting the polysaccharide in the present invention include ketotetrose such as erythrulose and the like; aldotetrose such as erythrose, threose and the like; ketopentose such as ribulose, xylulose and the like; aldopentose such as ribose, arabinose, xylose, lyxose and the like; ketohexose such as psicose (allulose), fructose, sorbose, tagatose and the like; aldohexose such as allose, altrose, glucose, mannose, gulose, idose, galactose, talose and the like; tetrose - heptose such as ketoheptose (e.g., sedoheptulose) and the like, deoxy sugar such as deoxyribose, fucose, fuculose, rhamnose and the like; uronic acid such as arabinoic acid, fructuronic acid, tagaturonic acid, glucuronic acid, galacturonic acid, mannuronic acid, iduronic acid, guluronic acid and the like; amino sugar such as glucosamine, N-acetylglucosamine, galactosamine, N-acetylgalactosamine, mannosamine, N-acetylmannosamine, N-acetylmuramic acid, neuraminic acid, N-acetylneuraminic acid and the like and N-acetylated compounds thereof, and the like.

As the polysaccharide in the present invention, homopolysaccharides, heteropolysaccharides, mucopolysaccharides composed of one kind or two or more kinds selected from the above-mentioned monosaccharides, and chemically modified compounds thereof such as deacetylated compounds, sulfated compounds and the like, and the like are used.

As the above-mentioned polysaccharides, those having various molecular weights from low molecular weight to high molecular weight can be used for the purpose of the present invention.

In the present specification, the "molecular weight" is a molecular weight measured by size-exclusion chromatography.

For the purpose of the present invention, it is preferable to use, as the above-mentioned polysaccharides, a polysaccharide having a weight average molecular weight of about 300 to 500,000, more preferably about 1,000 to 50,000, further preferably about 4,000 to 50,000, as measured by size-exclusion chromatography.

In the present specification, the above-mentioned weight average molecular weight may be simply indicated as "average molecular weight".

The size-exclusion chromatography of polysaccharides can be performed in elution conditions using a column using a hydrophilic polymer generally used as a carrier and a neutral salt eluent such as nitric acid sodium aqueous solution, according to the kind of polysaccharide and the like.

In the present invention, when the above-mentioned polysaccharide is the below-mentioned anionic polysaccharide, it can also be used in a salt form. Examples of the salt include alkali metal salt such as lithium salt, sodium salt, potassium salt and the like; alkaline earth metal salt such as magnesium salt, calcium salt and the like; ammonium salt; organic amine salt such as triethanolamine salt, pyridinium salt and the like, and the like.

For the purpose of the present invention, an anionic polysaccharide having a negatively-charged functional group is preferably used and, for example, polysaccharide containing uronic acid which has a carboxylic acid in a molecule, as a constituent unit, such as hyaluronic acid, polygalacturonic acid, pectin, alginic acid and the like; sulfated polysaccharides such as carrageenan, fucoidan, heparin, heparan sulfate, dextran sulfate (excluding those having a molecular weight of 4,000 kDa to 40,000 kDa), dermatan sulfate, keratan sulfate, chondroitin sulfate (chondroitin 4-sulfate, chondroitin 6-sulfate etc.) and the like are recited as preferable polysaccharides.

Among these, sulfated polysaccharide is more preferably used, and heparin, dextran sulfate (excluding those having a molecular weight of 4,000 kDa to 40,000 kDa), chondroitin sulfate and the like with a high degree of sufation are further preferably used. As the sulfated polysaccharide, one with a degree of sufation of all hydroxyl groups of about 10% to 90% is preferably used, and one with about 20% to 80% is more preferably used.

Dextran sulfate is composed only of glucose, and is the polysaccharide in which many of α-1,6-bonds are sulfated.

In the present invention, dextran sulfate other than those having a high molecular weight of 4,000 kDa to 40,000 kDa is used. Preferably, dextran sulfate having a weight average molecular weight of about 1,000 to 50,000, more preferably about 4,000 to 50,000, as measured by size-exclusion chromatography, is used.

As the additive of the present invention, one kind of the above-mentioned polysaccharides in the present invention may be selected and used, or two or more kinds thereof can also be selected and used in combination.

The content of the polysaccharide in the present invention in the additive for culture of the present invention is set so that the content of the polysaccharide in the present invention in the medium composition when added to the medium will fall within the range of the below-mentioned content.

From the aspects of cell aggregate formation-promoting effect and the like in the culture of stem cells, particularly preferable examples of the above-mentioned polysaccharides in the present invention include heparin and a salt thereof, and dextran sulfate having an average molecular weight (weight average molecular weight measured by size-exclusion chromatography) of about 5,000 to 50,000 and a salt thereof.

Therefore, in a particularly preferred embodiment of the present invention, one kind or two or more kinds selected from the group consisting of heparin and a salt thereof, and dextran sulfate having an average molecular weight of about 5,000 to 50,000 and a salt thereof is/are used as the polysaccharides in the above-mentioned present invention.

In the present invention, the above-mentioned polysaccharide in the present invention may be used as it is as an additive for culture, or may be dissolved or dispersed in a solvent such as water or the like and used as a liquid additive for culture such as aqueous solution, dispersion or the like, or may be mixed with a component generally used for formulation such as excipient, binder and the like and used as a solid additive for culture such as powder, granule, tablet or the like.

In addition, the above-mentioned polysaccharide in the present invention may be mixed with a part of the medium components described below such as carbohydrate, inorganic salt and the like and prepared as a culture additive.

From the viewpoint that the addition to a medium for culturing stem cells is convenient and blending with a medium is easy, the additive of the present invention is preferably provided in the form of liquid, powder, granule, tablet or the like.

The additive of the present invention is preferably prepared through a sterilization treatment. The method of the sterilization treatment is not particularly limited, and examples thereof include autoclave sterilization at 121°C for 20 min, radiation sterilization, ethylene oxide gas sterilization, filter filtration sterilization, and the like. The method can be appropriately selected according to the form and the like of the additive of the present invention.

The additive of the present invention is added to the components of the below-mentioned medium for culturing stem cells, and used for preparation of a medium for culturing stem cells, or used by adding to the below-mentioned medium for culturing stem cells.

When the additive of the present invention is added to a medium for culture and stem cells are cultured in suspension, the proliferation rate and the survival rate of the stem cells are improved, cell aggregates with controlled size and shape can be efficiently formed, and further, the rate of maintaining an undifferentiated state of stem cells is improved.

As used herein, the "cell aggregate" refers to a spherical cell assembly in which the cells are gathered together or aggregated. It is also referred to as a "spheroid". That the "cell aggregates with controlled size and shape are efficiently formed" means that small, spherical, and uniform cell aggregates can be formed at high density.

The present invention also provides a medium for culturing stem cells (hereinafter to be also referred to as "the medium of the present invention" in the present specification).

The medium of the present invention contains the polysaccharide in the present invention together with medium components generally used for culturing stem cells.

The medium of the present invention can contain only one kind or two or more kinds of the polysaccharide in the present invention in combination.

The polysaccharide in the present invention to be contained in the medium of the present invention may be contained in the form prepared as the above-mentioned additive of the present invention and together with the aforementioned medium component, or may be directly added to the medium component.

The content of the above-mentioned polysaccharides in the present invention in the medium of the present invention is generally 1 µg/mL to 1 mg/mL, preferably 10 µg/mL to 1 mg/mL, more preferably 20 µg/mL to 250 µg/mL, as the final concentration during culturing.

Examples of the medium component that can be contained in the medium of the present invention include medium components generally used for culturing stem cells. For example, sugar such as glucose, fructose, sucrose, maltose and the like; amino acid such as asparagine, aspartic acid, glutamine, glutamic acid and the like; protein and peptide such as albumin, transferrin and the like; serum; vitamin such as vitamin A, vitamin B groups (thiamine, riboflavin, pyridoxin, cyanocobalamin, biotin, folic acid, pantothenic acid, nicotine amide etc.), vitamin C, vitamin E and the like; fatty acid such as oleic acid, arachidonic acid, linoleic acid and the like, lipid such as cholesterol and the like; inorganic salt such as sodium chloride, potassium chloride, calcium chloride, magnesium sulfate, sodium dihydrogen phosphate and the like; trace element such as zinc, copper, selenium and the like; buffering agent such as N,N-bis(2-hydroxyethyl)-2-aminoethanesulfonic acid (N,N-bis(2-hydroxyethyl)-2-aminoethanesulfonic acid (BES)), 4-(2-hydroxyethyl)-1-piperazineethanesulfonic acid (4-(2-hydroxyethyl)-1-piperazineethanesulfonic acid (HEPES)), N-[tris (hydroxymethyl)methyl]glycine (N-[tris(hydroxymethyl)methyl]glycine (Tricine)) and the like; antibiotic such as amphotericin B, kanamycin, gentamicin, streptomycin, penicillin and the like; cell adhesion factor and extracellular matrix component such as Type I collagen, Type II collagen, fibronectin, laminin, poly-L-lysine, poly-D-lysine and the like; cytokine and growth factor such as interleukin, fibroblast growth factor (FGF), hepatocyte growth factor (HGF), transforming growth factor (TGF)-α, transforming growth factor (TGF)-β, vascular endothelium growth factor (VEGF), activin A and the like; hormone such as dexamethasone, hydrocortisone, estra diol, progesterone, glucagon, insulin and the like, and the like can be mentioned. An appropriate component can be selected and used according to the type of the stem cells to be cultured.

Since serum may contain unidentified factor, prion, virus and the like, it is preferable that the medium of the present invention be free of a serum in the medium component. In addition, when the medium of the present invention is prepared as a medium for culturing human stem cells, it is preferable that the medium be free of a component derived from an animal other than human.

In the present invention, an existing medium for culturing stem cells can be used as a medium component, and a commercially available medium can also be used.

Examples of the medium include STEMPRO (registered trade mark) hESC SFM medium (Life Technologies), mTeSR1 medium (STEMCELL Technologies), TeSR2 medium (STEMCELL Technologies), TeSR-E8 medium (STEMCELL Technologies), Essential 8 medium (Life Technologies), HEScGRO (trade mark) Serum-Free medium for hES cells (Millipore), PluriSTEM (trade mark) Human ES/iPS medium (EMD Millipore), NutriStem (registered trade mark) hESC XF medium (Biological Industries Israel Beit-Haemek Ltd., NutriStem (trade mark) XF/FF Culture medium (Stemgent), AF NutriStem (registered trade mark) hESC XF medium (Biological Industries Israel Beit-Haemek Ltd., S-medium (DS pharma biomedical), StemFit (registered trade mark) AK03 medium (Ajinomoto Co., Inc.), hESF9 medium, hESF-FX medium, CDM medium, DEF-CS 500 Xeno-Free 3D Spheroid Culture medium (Cellartis), StemFlex medium (Thermo Fisher Scientific) and the like.

For the purpose of the present invention, a feeder-free medium for culturing stem cells is preferably used, and a serum-free medium is more preferably used. In addition, a medium for culturing human stem cells preferably does not contain a component derived from an animal other than human (xeno-free medium).

From the aspect that it is used for suspension culture of stem cells, the medium of the present invention is preferably in the form of a liquid such as solution, dispersion or the like.

The medium of the present invention can be prepared by adding a component appropriately selected from the above-mentioned medium components together with the polysaccharide in the present invention to a solvent such as water and the like according to a known composition, and dissolving or dispersing them.

The medium of the present invention can also be prepared by adding the polysaccharide in the present invention to the above-mentioned medium for culturing stem cells which is provided by each company or institution, and dissolving or dispersing them.

Furthermore, the medium of the present invention can also be prepared by concentrating than the concentration at the time of use, preparing a freeze-dried powder, using same by diluting with a solvent such as water and the like, or using same by dissolving or dispersing in a solvent such as water and the like.

The medium of the present invention is preferably prepared by applying a sterilization treatment as mentioned above.

Suspension culture of stem cells using the medium of the present invention makes it possible to perform three dimensional culture of stem cells with a high proliferation rate and a high survival rate, and to efficiently form a cell aggregate with controlled size and shape. In addition, the rate of maintaining an undifferentiated state of stem cells can be improved.

Furthermore, the present invention provides a method for culturing stem cells (hereinafter to be also referred to as "the culture method of the present invention" in the present specification).

The culture method of the present invention includes suspension culturing stem cells in a medium for culturing stem cells containing the polysaccharide in the present invention.

The "medium for culturing stem cells containing the polysaccharide in the present invention" is as described above. The polysaccharide in the present invention which is contained in the medium for culturing stem cells in the present invention may be one prepared and added as the above-mentioned additive of the present invention, or the polysaccharide in the present invention may be directly added.

In the present invention, the polysaccharide in the present invention is added to the medium such that the final concentration at the time of culture would be generally 1 µg/mL to 1 mg/mL, preferably 10 µg/mL to 1 mg/mL, more preferably 20 µg/mL to 250 µg/mL.

In the culture method of the present invention, the stem cells can be cultured according to a general method for suspension culture. That is, using a culture device or culture apparatus such as a cell culture plate, a cell culture flask, a bioreactor or the like as appropriate according to the culture scale, stem cells are seeded in the above-mentioned medium of the present invention or a medium for culturing stem cells added with the additive of the present invention and cultured at generally 25°C to 39°C, preferably 33°C to 39°C, in the presence of generally 4% by volume to 10% by volume, preferably 4% by volume to 6% by volume, of carbon dioxide, and in the presence of generally 1% by volume to 25% by volume, preferably 4% by volume to 20% by volume, of oxygen for generally 1 day to 30 days, preferably 3 days to 14 days. The medium is exchanged every 2 to 3 days.

To exchange the medium, the stem cells and the medium may be separated by centrifugation or filtration, and then a new medium may be added to the stem cells. Alternatively, stem cells may be appropriately concentrated by centrifugation or filtration, and then a new medium may be added to the concentrate.

The gravity acceleration (G) during the above-mentioned centrifugation is generally 50G to 1,000G, preferably 100G to 500G, and the size of the fine pores in the filter to be used for filtration is generally 10 µm to 200 µm.

To efficiently obtain a cell aggregate having a controlled size, it is preferable to culture the stem cells with stirring or shaking.

Stirring is performed at a stirring rate of generally 10 rpm to 2,000 rpm, preferably 40 rpm to 1,000 rpm.

Shaking is performed at a shaking rate of generally 10 rpm to 500 rpm, preferably 50 rpm to 250 rpm.

The cultured stem cells can be recovered by centrifugation or filtration using a filter.

Centrifugation is performed at 50 G to 1,000 G, preferably 100 G to 500 G, for about 1 min to 10 min.

Filtration can be performed using a filter with fine pores of about 10 µm to 200 µm.

The cultured stem cells are preferably preserved using a freezing medium containing a cryoprotective agent such as STEM-CELLBANKER (Nippon Zenyaku Kogyo Co., Ltd.) and the like in liquid nitrogen.

According to the culture method of the present invention, stem cells can be three-dimensionally cultured at a high proliferation rate and a high survival rate, and a cell aggregate having a controlled size and shape can be efficiently obtained. In addition, cultured cells of stem cells with an improved rate of maintaining an undifferentiated state can be obtained.

### [Example]

The present invention is explained in more detail in the following by referring to Examples.

In the following Examples, using a medium for culturing stem cells shown below, the following polysaccharides as the polysaccharide in the present invention, and undifferentiated human iPS cell (hiPSC) as the stem cell, suspension culture by stirring and suspension culture by shaking were performed as shown below.
(1) As the media for culturing stem cells, StemFit (registered trade mark) AK03N medium (Ajinomoto Co., Inc.), Essential 8 medium (Thermo Fisher Scientific, A1517001), mTeSR1 medium (STEMCELL Technologies, 85850), DEF-CS 500 Xeno-Free 3D Spheroid Culture medium (Cellartis, Y30047), and StemFlex medium (Thermo Fisher Scientific, A3349401) were used.
(2) As the polysaccharide in the present invention, heparin sodium (Nacalai Tesque, 17513-54), heparin lithium (Nacalai Tesque, 02869-74), heparin ammonium (Sigma-Aldrich Co. LLC., H6279), clexane (enoxaparin sodium) (Sanofi K.K.) (low-molecular-weight heparin sodium with average molecular weight=4,500), heparan sulfate (produced according to the method described in WO 2017/115675), and sodium dextran sulfate (Wako Pure Chemical Industries, Ltd., 191-08365) (average molecular weight=5,000), and sodium dextran sulfate (MP BIOMEDICALS, 0216011090) (molecular weight 36,000 to 50,000) were used.
(3) As the undifferentiated hiPSC, hiPS cells of 1210B2 strain and 1231A3 strain (see Nakagawa, M. et al., Sci. Rep. 4, 3594, 2014) were used.

(4) Non-adherent cell culture by stirring was performed using single-use bioreactors 30 mL volume (ABLE Corporation, BWV-S03A) and 5 mL volume (ABLE Corporation, S-1467) as culture vessels.

For 30 mL-scale suspension culture, a medium (30 mL) containing 10 µM Rho-associated kinase inhibitor (Y-27632) (Fujifilm Wako Pure Chemical Industries, Ltd., 034-24024) was added to a 30 mL volume bioreactor; for 5 mL-scale suspension culture, a medium (5 mL) containing 10 µM Rho-associated kinase inhibitor (Y-27632) was added to a 5 mL volume bioreactor, single-celled hiPSCs were added, and stirring culture was performed under conditions of 37°C, 5% by volume carbon dioxide at a rotating speed described in each Example.

The medium was exchanged from day 2 and thereafter. The medium was exchanged by extracting the medium supernatant in the amount indicated in each Example, centrifuging same at 500G for 5 min, removing the supernatant, adding the same amount of a fresh medium, suspending pellets and adding the suspension to the bioreactor.

Suspension culture by shaking was performed by adding a medium (5 mL) containing 10 µM Rho-associated kinase inhibitor (Y-27632) to a 6-well cell culture plate (Greiner Bio-One International, 657160), adding single-celled hiPSC at 1x10⁶ cells, and horizontally shaking under the conditions of 37°C, 5% by volume carbon dioxide at 95 rpm.

In each of the following Examples, the measurement of the number of cell aggregates and their major axis, the measurement of cell number and survival rate, and the measurement of the rate of maintaining an undifferentiated state in the cultured stem cells were performed as described below.

### (1) Measurement of the number of cell aggregates and their major axis

The medium supernatant containing the cell aggregates (500 µl) was collected on a 24-well plate. The cell aggregates were dispersed by shaking, and the entire well was photographed with a BZ-X fluorescence microscope (Keyence). By macrocell counting on the obtained image, the average number and average major axis of the cell aggregates were determined.

### (2) Measurement of cell number and survival rate

The total amount of the medium supernatant containing the cell aggregates was recovered, and centrifuged at 500 G for 5 min. After removing the supernatant, tapping was performed 10 times, 1 mL of cell separation/dispersion solution (Accumax (Millipore, SCR006)) was added, and the cell aggregate pellet was suspended. After incubating for 5 min at room temperature, the cell aggregate was resuspended by pipetting. After incubating again for 5 min at room temperature, the cell aggregate was single-celled by pipetting. The medium (4 mL) was added and the mixture was centrifuged at 500 G for 5 min. After removing the supernatant, the pellets were disrupted by tapping 10 times. The cells were resuspended by adding a medium containing 1 mL of Rho-associated kinase inhibitor (Y-27632) and pipetting. The cell suspension was passed through a 40 µm cell strainer (BD Falcon (Corning Incorporated), 2-1919-02), and the cell strainer was prewashed with a medium containing 4 mL of a Rho-associated kinase inhibitor (Y-27632). The number of cells and the survival rate were measured by analyzing the collected cell suspension with a viable/dead cell autoanalyzer Vi-CELL XR (Beckman Coulter).

### (3) Measurement of rate of maintaining an undifferentiated state

The cells single-celled after culturing were immobilized with a cell immobilization/cell permealization solution (BD Cytofix/Cytoperm (trade mark) Kit (BD Biosciences, 554714)). Specifically, 200 µL of Cytofix/Cytoperm was added, and the hiPSCs were allowed to stand on ice for 20 min to fix them.

Then, 1 mL of BD Permanent wave/Wash buffer (trade mark) (BD Biosciences, 554723) was added, and the mixture was centrifuged at 5,000 rpm for 2 min to remove the supernatant. Then, it was suspended in an adequate amount of BD Perm/Wash buffer (trade mark), a sample for double staining, a sample for single staining, a sample for isotype control, and a sample for non-staining were each separately dispensed into a centrifuge tube, centrifuged at 5,000 rpm for 2 min, and the supernatant was removed.

Double staining and single staining were performed by adding 100 µL of a solution obtained by adding one or both of 1:5 (5-fold) diluted Alexa Fluor (registered trade mark) 488 mouse anti-oct3/4 (Becton Dickinson, 560253) and 1:10 (10-fold) diluted Alexa Fluor (registered trade mark) 647 mouse anti-SSEA-4 (Becton Dickinson, 560796) to BD Perm/Wash buffer (trade mark), and incubating at room temperature under shading for 20 min.

To the isotype control sample was added 100 µL of BD Perm/Wash buffer (trade mark) added with 1:20 (20-fold) diluted Alexa Fluor (registered trade mark) 488 Mouse IgG1 κ Isotype Control (Becton Dickinson, 557721) or 1:20 (20-fold) diluted Alexa Fluor (registered trade mark) 647 Mouse IgG3, κ Isotype Control (Becton Dickinson, 560803), and the mixture was incubated similarly at room temperature under shading for 20 min.

After each of the above-mentioned reactions, 500 µL of BD Perm/Wash buffer (trade mark) was added, and the mixture was centrifuged at 5,000 rpm for 2 min to remove the supernatant. To each sample was added 1 mL of Focusing fluid (Thermo Fisher Scientific, 4488621), the mixture was centrifuged again at 5,000 rpm for 2 min and suspended in 200 µL of Focusing fluid (Thermo Fisher Scientific, 4488621). The prepared samples were analyzed by Attune NxT Flow Cytometer (Thermo Fisher Scientific). Alexa Fluor (registered trade mark) 488 dye was detected by BL1, and Alexa Fluor (registered trade mark) 647 dye was detected by RL1.

The rate of maintaining an undifferentiated state of cell can be shown by an Oct3/4/SSEA4 positive rate of the cultured cells.

### [Example 1] Stirring culture of hiPSC using medium containing heparin sodium and sodium dextran sulfate (average molecular weight=5,000)

To StemFit (registered trade mark) AK03N medium were respectively added 0.25 mg/mL heparin sodium and 0.1 mg/mL sodium dextran sulfate (average molecular weight =5,000), hiPSC was suspension cultured by stirring, and the effects of sodium heparin and sodium dextran sulfate with an average molecular weight of 5,000 on the formation of cell aggregates and cell state of hiPSC were evaluated.

The 1210B2 strain of hiPSC was seeded at 2x10⁶ cells in a 30 mL bioreactor and cultured with stirring at a stirring rate of 55 rpm. On days 2 and 3, 21 mL of the medium was exchanged.

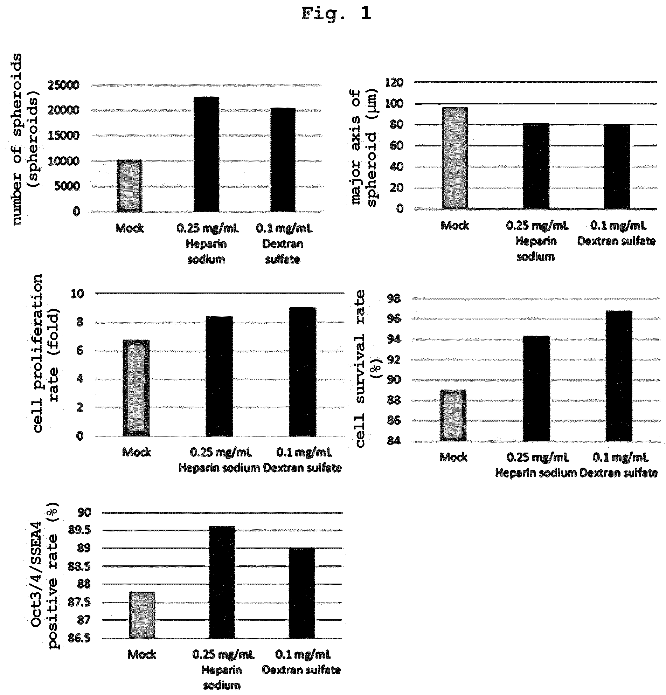

On day 4, the number of cell aggregates formed, the average major axis of the cell aggregates, the proliferation rate, the cell survival rate, and the rate of maintaining an undifferentiated state were measured by the above-mentioned methods. The results are shown in Fig. 1.

As shown in Fig. 1, when cultivated in a medium supplemented with heparin sodium or sodium dextran sulfate (average molecular weight=5,000), the number of cell aggregates formed was approximately doubled as compared with no addition of these (Mock). It was found that the major axis of the cell aggregates decreased. In addition, the cell proliferation rate, survival rate, and the rate of maintaining an undifferentiated state were all improved.

From these results, it was clarified that stirring culture of hiPSC in a medium supplemented with heparin sodium or sodium dextran sulfate with an average molecular weight of 5,000 enables formation of a large amount of small cell aggregates, and efficient proliferation of iPS cells with high survival rate and high rate of maintaining an undifferentiated state.

### [Example 2] Stirring passage culture of hiPSC using medium containing heparin sodium

To StemFit (registered trade mark) AK03N medium was added 0.1 mg/mL heparin sodium, hiPSC was cultured with stirring for 4 passages, and the effects of heparin sodium when passage was repeated were evaluated.

The 1210B2 strain of hiPSC was seeded at 6x10⁶ cells in a 30 mL bioreactor and cultured with stirring at a stirring rate of 120 rpm. On days 2 - 5, 9 - 11, 15 - 17, and 21, 21 mL of the medium was exchanged and the total amount 30 mL of the medium was exchanged on days 6, 12, 18, and 22. On days 7, 13, 19, and 23, the cell aggregates were disrupted, and the number of cell aggregates formed, the average major axis of the cell aggregates, cell proliferation rate (accumulated proliferation rate), cell survival rate, and the rate of maintaining an undifferentiated state were measured. The recovered and single-celled cells were subcultured into a new bioreactor at 6x10⁶ cells on days 7 and 13, and at 3x10⁷ cells on day 19.

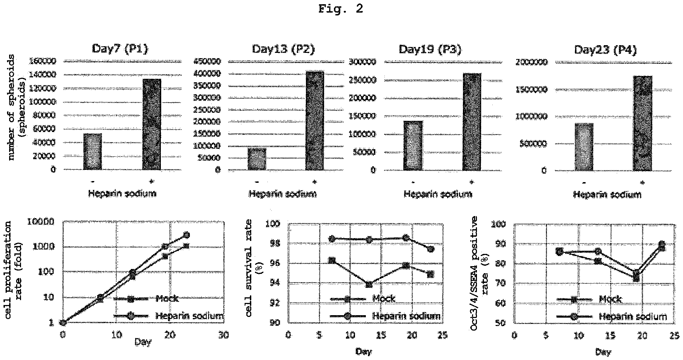

The number of cell aggregates, cell proliferation rate, cell survival rate, and the rate of maintaining an undifferentiated state in each passage are shown in Fig. 2.

As is clear from Fig. 2, when cultured in a medium supplemented with heparin sodium, the number of cell aggregates formed, cell proliferation rate, survival rate, and the rate of maintaining an undifferentiated state were all improved in any passage number as compared with a medium without addition of heparin sodium (Mock).

From these results, it was clarified that stirring culture of hiPSC in a medium supplemented with heparin sodium enables continuous formation of a large amount of small cell aggregates, and efficient proliferation of iPS cells with high survival rate and high rate of maintaining an undifferentiated state.

### [Example 3] Stirring passage culture of hiPSC using medium containing sodium dextran sulfate

To StemFit (registered trade mark) AK03N medium was added 0.1 mg/mL sodium dextran sulfate (average molecular weight=5,000), hiPSC was cultured with stirring for 3 passages, and the effects of sodium dextran sulfate (average molecular weight=5,000) when passage was repeated were evaluated.

The 1210B2 strain of hiPSC was seeded at 6x10⁶ cells in a 30 mL bioreactor and cultured with stirring at a stirring rate of 120 rpm, and stirring culture was performed at a stirring rate of 55 rpm for 8 days, and at 120 rpm for 6 days thereafter. On days 2 - 3, 6 - 7, and 10 - 12, 21 mL of the medium was exchanged and the total amount 30 mL of the medium was exchanged on days 13 and 22. On days 4, 8 and 14, the cell aggregates were disrupted, and the number of cell aggregates formed, the average major axis of the cell aggregates, cell proliferation rate, cell survival rate, and the rate of maintaining an undifferentiated state were measured. The recovered and single-celled cells were subcultured into a new bioreactor at 6x10⁶ cells on days 4 and 8.

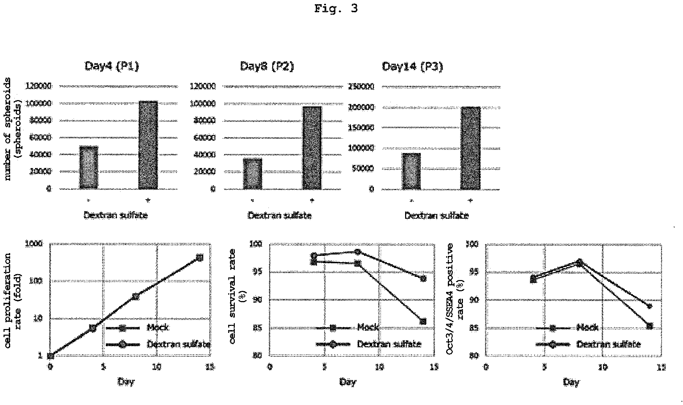

The number of cell aggregates, cell proliferation rate, cell survival rate, and the rate of maintaining an undifferentiated state in passage are shown in Fig. 3.

As shown in Fig. 3, when cultured in a medium supplemented with sodium dextran sulfate (average molecular weight=5,000), the cell proliferation rate did not change but the number of cell aggregates formed, cell survival rate, and the rate of maintaining an undifferentiated state were all improved in any passage number as compared with a medium without addition of sodium dextran sulfate (average molecular weight=5,000) .

From these results, it was clarified that stirring culture of hiPSC in a medium supplemented with sodium dextran sulfate with an average molecular weight of 5,000 enables continuous formation of a large amount of small cell aggregates, and efficient proliferation of iPS cells with high survival rate and high rate of maintaining an undifferentiated state.

### [Example 4] Stirring culture of hiPSC using medium containing various heparins

To StemFit (registered trade mark) AK03N medium were respectively added 0.25 mg/mL heparin sodium (Na), heparin lithium (Li), heparin ammonium (NH₄), heparan sulfate and 50 U/mL clexane (enoxaparin sodium) (low-molecular-weight heparin; average molecular weight=4,500), hiPSC was cultured with stirring, and the effects of various heparins were evaluated.

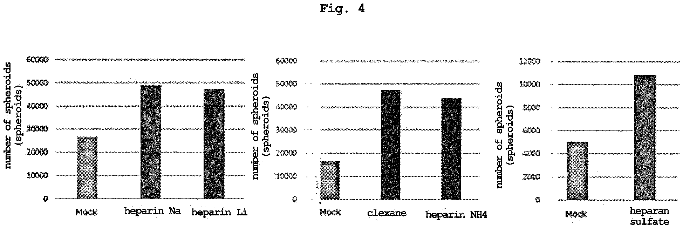

The 1210B2 strain of hiPSC was seeded at 1x10⁶ cells in a 5 mL bioreactor and cultured with stirring at a stirring rate of 120 rpm. From day 2, 3.5 mL of the medium was exchanged every day, and the number of cell aggregates formed was measured on day 3 when a medium supplemented with heparin Na and heparin Li was used and on day 4 when a medium supplemented with each of the other heparins was used. The results are shown in Fig. 4.

As shown in Fig. 4, when stirring culture was performed using a medium supplemented with each heparin salt, low-molecular-weight heparin and heparan sulfate, the number of cell aggregates formed was improved as compared to Mock without addition of any of the above.

From these results, it was clarified that heparins different in the kind of counter cation, molecular weight or degree of sufation have the effect of forming a large amount of the cell aggregates.

### [Example 5] Stirring culture of hiPSC using medium containing sodium dextran sulfate with different molecular weight

To StemFit (registered trade mark) AK03N medium were respectively added sodium dextran sulfate having an average molecular weight of 5,000 and sodium dextran sulfate having an molecular weight of 36,000 to 50,000 each at 0.1 mg/mL, hiPSC was cultured with stirring, and the effects of sodium dextran sulfate having different molecular weight were evaluated.

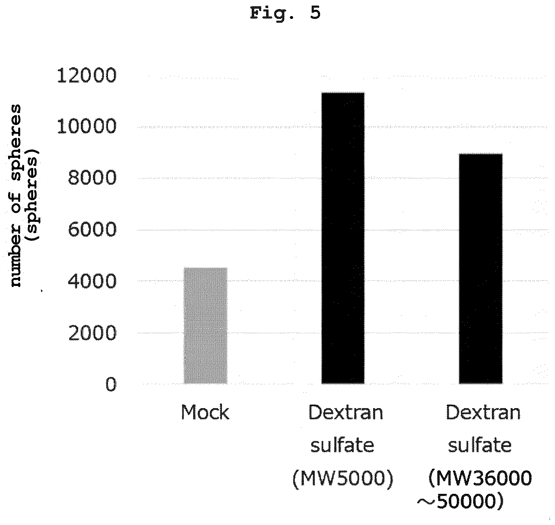

The 1210B2 strain of hiPSC was seeded at 1x10⁶ cells in a 5 mL bioreactor, and cultured with stirring at a stirring rate of 120 rpm, and the number of cell aggregates formed was measured on day 4. The results are shown in Fig. 5.

As shown in Fig. 5, the addition of sodium dextran sulfate with any molecular weight improved the number of cell aggregates formed as compared to Mock without addition of sodium dextran sulfate. Addition of sodium dextran sulfate with an average molecular weight of 5,000 showed a high cell aggregate formation-promoting effect.

### [Example 6] Stirring culture of hiPSC using various media containing heparin sodium

To each of StemFlex medium, DEF-CS 500 Xeno-Free 3D Spheroid Culture medium, Essential 8 medium, and mTeSR1 medium was added 0.25 mg/mL heparin sodium, hiPSC was cultured with stirring, and the effects of heparin sodium in the stirring culture using each of the aforementioned media were evaluated.

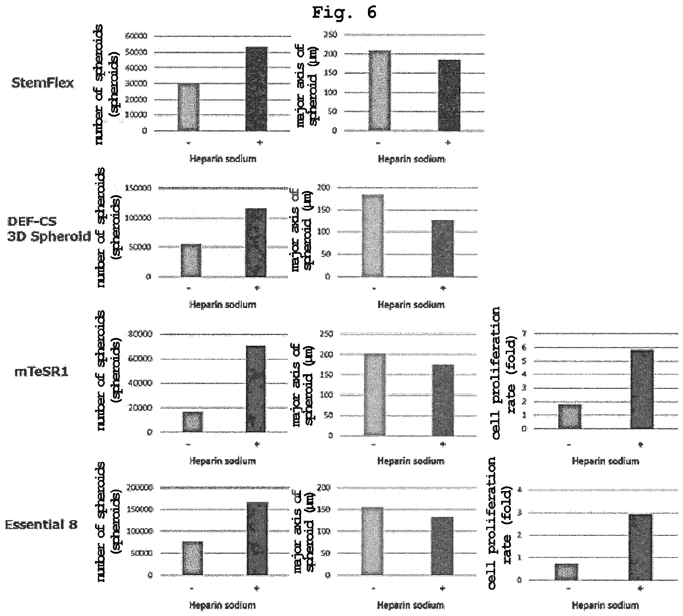

The 1210B2 strain of hiPSC was seeded at 6x10⁶ cells in a 30 mL bioreactor, and cultured with stirring at a stirring rate of 55 rpm. From day 2 of culture, 21 mL of the medium was exchanged every day, and the number of cell aggregates formed, the major axis of the cell aggregates, and cell proliferation rate were measured on day 6 in the experiment using DEF-CS 500 Xeno-Free 3D Spheroid Culture Medium and on day 5 in other experiments. The results are shown in Fig. 6.

As shown in Fig. 6, when heparin sodium was added to any of the media, the number of cell aggregates formed was improved, and the major axis of the cell aggregates decreased as compared with no addition of heparin sodium. In culture using mTeSR1 medium and Essential 8 medium, addition of heparin sodium remarkably improved the cell proliferation rate.

From these results, it was clarified that the heparin salt has the effects of improving the cell aggregate forming rate, controlling the cell size, and promoting proliferation, irrespective of the kind of the medium used for the culture.

### [Example 7] Stirring culture of hiPSC using various media containing sodium dextran sulfate (average molecular weight=5,000)

To each of essential 8 medium and mTeSR1 medium was added 0.1 mg/mL sodium dextran sulfate (average molecular weight=5,000), hiPSC was cultured with stirring, and the effects of sodium dextran sulfate (average molecular weight =5,000) in each of the aforementioned media were evaluated.

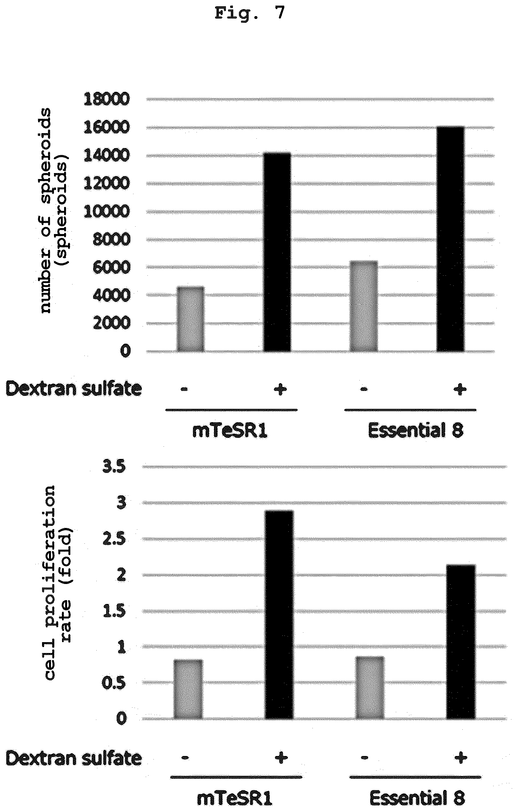

The 1210B2 strain of hiPSC was seeded at 1x10⁶ cells in a 5 mL bioreactor and cultured with stirring at a stirring rate of 80 rpm. From day 2, 3.5 mL of the medium was exchanged every day, and the number of cell aggregates formed and cell proliferation rate were measured on day 4. The results are shown in Fig. 7.

As shown in Fig. 7, in any medium, when cultured by adding sodium dextran sulfate (average molecular weight=5,000), the number of cell aggregates formed was improved and the cell proliferation rate was markedly improved.

From these results, it was clarified that sodium dextran sulfate with an average molecular weight of 5,000 promotes formation and proliferation of cell aggregates, irrespective of the kind of the medium used.

### [Example 8] Stirring culture of hiPSC using medium containing various concentrations of heparin sodium

To mTeSR1 medium was added 0.25 µg/mL to 250 µg/mL heparin sodium, hiPSC was cultured with stirring, and the concentration dependency of heparin sodium in the cell aggregate forming rate-improving effect was evaluated.

The 1210B2 strain of hiPSC was seeded at 1x10⁶ cells in a 5 mL bioreactor, and stirring culture was performed at a stirring rate of 80 rpm. On days 2 - 3 of culture, 3.5 mL of the medium was exchanged, and the number of cell aggregates formed was measured on day 4.

In addition, to StemFit (registered trade mark) AK03N medium was added heparin sodium at a concentration of 0.1 mg/mL or 1 mg/mL, and hiPSC was cultured with stirring.

The 1210B2 strain of hiPSC was seeded at 6x10⁶ cells in a 30 mL bioreactor, and stirring culture was performed at a stirring rate of 120 rpm. On days 2 - 4 of culture, 21 mL of the medium was exchanged, 30 mL of the medium was exchanged on day 5, and the number of cell aggregates formed was measured on day 6.

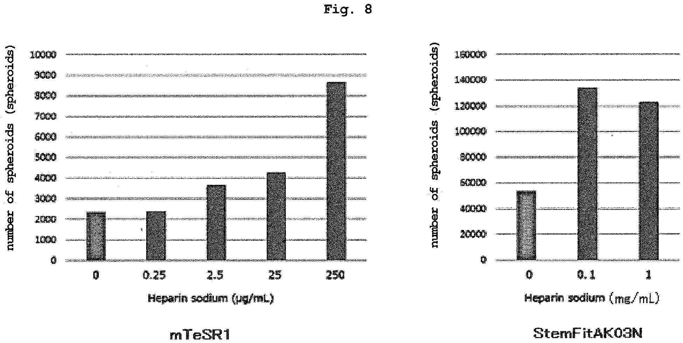

In each of the above, the number of cell aggregates was measured and the results are shown in Fig. 8.

As shown in Fig. 8, when cultured by adding 2.5 µg/mL to 250 µg/mL heparin sodium to the mTeSR1 medium, the number of cell aggregates formed was improved, and the cell proliferation rate was markedly improved. Also, when stirring culture was performed by adding heparin sodium at a concentration of 0.1 mg/mL or 1 mg/mL to the StemFit (registered trade mark) AK03N medium, the cell aggregate forming rate was drastically improved.

From the above-mentioned results, it was clarified that addition of about 1 µg/mL to about 1 mg/mL heparin sodium achieves the effect of improving the cell aggregate forming rate.

### [Example 9] Stirring culture of hiPSC using medium containing various concentrations of sodium dextran sulfate (average molecular weight=5,000)

To mTeSR1 medium was added 0.1 µg/mL to 1000 pg/mL sodium dextran sulfate (average molecular weight=5,000), hiPSC was cultured with stirring, and the concentration dependency of sodium dextran sulfate (average molecular weight=5,000) in the cell aggregate forming rate-improving effect was evaluated.

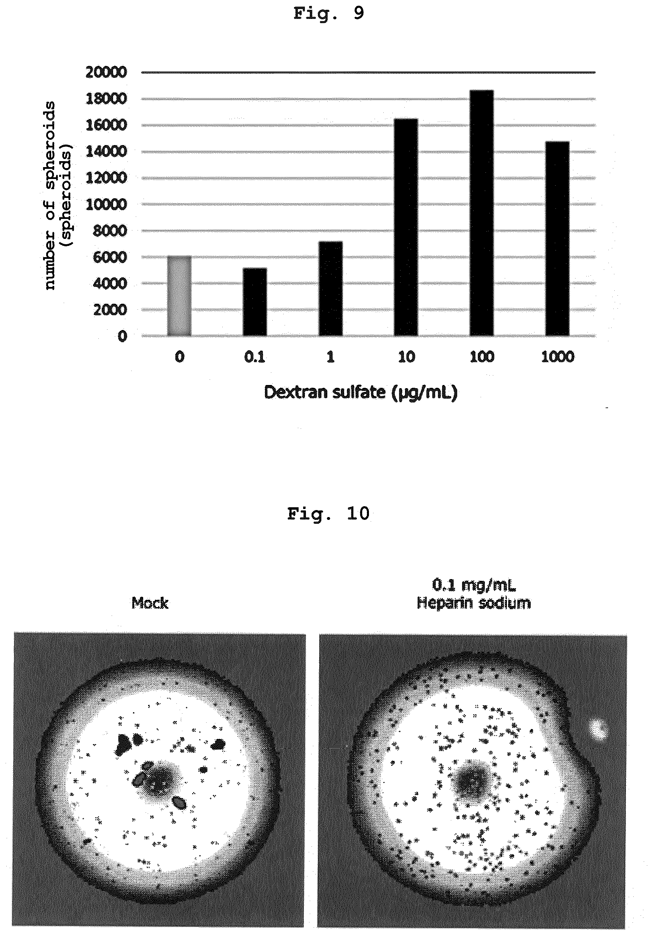

The 1210B2 strain of hiPSC was seeded at 1x10⁶ cells in a 5 mL bioreactor, and stirring culture was performed at a stirring rate of 80 rpm. On days 2 and 3 of culture, 3.5 mL of the medium was exchanged, and the number of cell aggregates formed was measured on day 4. The results are shown in Fig. 9.

As shown in Fig. 9, when stirring culture was performed using a medium supplemented with 10 µg/mL to 1000 µmL sodium dextran sulfate (average molecular weight=5,000), remarkable improvement in the number of cell aggregates formed was observed.

From these results, it was clarified that addition of sodium dextran sulfate with an average molecular weight of 5,000 at a concentration of about 10 µg/mL to about 1 mg/mL achieves a cell aggregate forming rate-improving effect.

### [Example 10] Shaking culture of hiPSC using medium containing heparin sodium

To StemFit (registered trade mark) AK03N medium was added 0.1 mg/mL heparin sodium, hiPSC was cultured with shaking, and the effects of heparin sodium on the formation of cell aggregates and cell state of hiPSC were evaluated.

The 1210B2 strain of hiPSC was seeded at 1x10⁶ cells in the above-mentioned medium (5 mL) supplemented with 10 µM Rho-associated kinase inhibitor (Y-27632) in a 6-well cell culture plate, and shaking culture was performed at a shaking rate of 95 rpm. The observation results of the cultured cells on day 1 of culture under a BZ-X microscope are shown in Fig. 10.

As shown in Fig. 10, the group cultured without addition of heparin sodium (Mock) showed coagulated huge cell aggregates, whereas the group cultured by adding heparin sodium showed finely dispersed cell aggregates and formation of uniform cell aggregates.

From these results, it was clarified that shaking culture of hiPSC in a medium containing heparin sodium enables formation of uniform cell aggregates.

### [Example 11] Stirring culture of hiPSC using medium containing various concentrations of sodium dextran sulfate (average molecular weight=5,000)

To StemFit (registered trade mark) AK03N medium was added 10 µg/mL to 1000 µg/mL sodium dextran sulfate (average molecular weight=5,000), hiPSC was cultured with stirring, and the concentration dependency of sodium dextran sulfate (average molecular weight=5,000) in the cell aggregate forming rate-improving effect was evaluated.

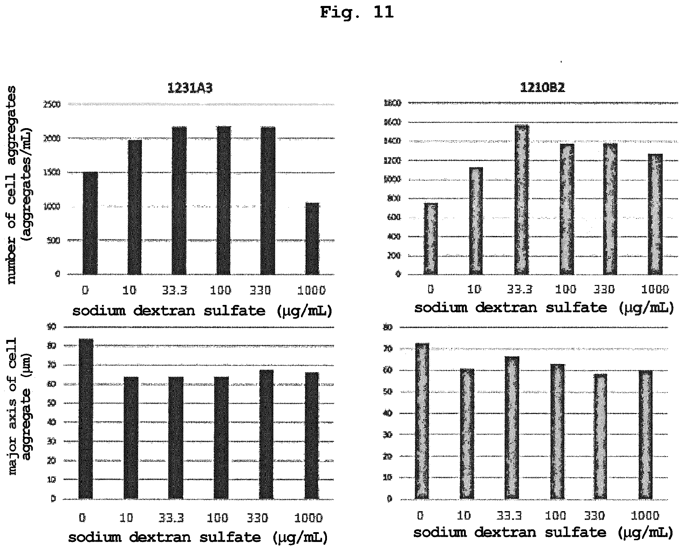

The 1231A3 strain and 1210B2 strain of hiPSC were each seeded at 1x10⁶ cells in a 5 mL bioreactor, and stirring culture was performed at a stirring rate of 80 rpm. The number of cell aggregates formed was measured on day 2. The results are shown in Fig. 11.

As shown in Fig. 11, when 1231A3 strain was used and stirring culture was performed using a medium supplemented with 10 µg/mL to 330 µg/mL sodium dextran sulfate (average molecular weight=5,000), an improvement in the number of cell aggregates formed was observed, and a decrease in the major axis of the cell aggregates was observed at a concentration of 10 µg/mL to 1000 µg/mL.

On the other hand, when 1210B2 strain was used and 10 µg/mL to 1000 µg/mL sodium dextran sulfate (average molecular weight=5,000) was added, an improvement in the number of cell aggregates formed and a decrease in the major axis of the cell aggregates were observed.

From these results, the possibility was suggested that the addition of sodium dextran sulfate with an average molecular weight of 5,000 at a concentration of about 10 µg/mL to about 1 mg/mL may achieve an improvement in the cell aggregate forming rate and the effect of controlling the size of the cell aggregates, irrespective of the type of the hiPSC strain.

### [Industrial Applicability]

As described in detail above, according to the present invention, an additive and a medium for culturing that are useful for suspension culture of stem cells can be provided.

The additive and the medium for culturing of the present invention can improve the proliferation rate and the survival rate of stem cells in suspension culture of the stem cells, improve the forming rate of cell aggregates with controlled size and shape, and further improve the rate of maintaining an undifferentiated state.

Furthermore, the present invention can provide a culture method of stem cells.

According to the culture method of the present invention, stem cells can be three-dimensionally cultured at a high proliferation rate and a high survival rate, and a cell aggregate having a controlled size and shape can be efficiently obtained. In addition, cultured cells of stem cells with an improved rate of maintaining an undifferentiated state can be obtained.

This application is based on a patent application No. 2018-122532 filed in Japan, the contents of which are incorporated in full herein.

## Claims

1. An additive for culturing stem cells, comprising a polysaccharide other than dextran sulfate having a molecular weight of 4,000 kDa to 40,000 kDa.

2. The additive according to claim 1, wherein the stem cell is one kind or two or more kinds selected from the group consisting of an adult stem cell, an embryonic stem cell and an induced pluripotent stem cell.

3. The additive according to claim 1 or 2, wherein the polysaccharide other than the dextran sulfate having a molecular weight of 4,000 kDa to 40,000 kDa is the aforementioned polysaccharide other than the dextran sulfate, and is an anionic polysaccharide having a negatively-charged functional group or a salt thereof.

4. The additive according to any one of claims 1 to 3, wherein the polysaccharide other than the dextran sulfate having a molecular weight of 4,000 kDa to 40,000 kDa is a sulfated polysaccharide other than the aforementioned dextran sulfate, or a salt thereof.

5. The additive according to any one of claims 1 to 4, wherein the polysaccharide other than the dextran sulfate having a molecular weight of 4,000 kDa to 40,000 kDa is one kind or two or more kinds selected from the group consisting of heparin and a salt thereof, and dextran sulfate having a weight average molecular weight of 5,000 to 50,000 and a salt thereof.

6. The additive according to any one of claims 1 to 5, wherein the additive is added to a medium for culturing stem cells.

7. The additive according to claim 6, wherein the additive is added such that a concentration of the polysaccharide other than the dextran sulfate having a molecular weight of 4,000 kDa to 40,000 kDa is 1 µg/mL to 1 mg/mL relative to the total amount of the medium.

8. A medium for culturing stem cells, comprising a polysaccharide other than dextran sulfate having a molecular weight of 4,000 kDa to 40,000 kDa.

9. The medium according to claim 8, wherein the medium is for culturing one kind or two or more kinds selected from the group consisting of an adult stem cell, an embryonic stem cell and an induced pluripotent stem cell.

10. The medium according to claim 8 or 9, wherein the polysaccharide other than the dextran sulfate having a molecular weight of 4,000 kDa to 40,000 kDa is the aforementioned polysaccharide other than the dextran sulfate, and is an anionic polysaccharide having a negatively-charged functional group or a salt thereof.

11. The medium according to any one of claims 8 to 10, wherein the polysaccharide other than the dextran sulfate having a molecular weight of 4,000 kDa to 40,000 kDa is a sulfated polysaccharide other than the aforementioned dextran sulfate, or a salt thereof.

12. The medium according to any one of claims 8 to 11, wherein the polysaccharide other than the dextran sulfate having a molecular weight of 4,000 kDa to 40,000 kDa is one kind or two or more kinds selected from the group consisting of heparin and a salt thereof, and dextran sulfate having a weight average molecular weight of 5,000 to 50,000 and a salt thereof.

13. The medium according to any one of claims 8 to 12, wherein a content of the polysaccharide other than dextran sulfate having a molecular weight of 4,000 kDa to 40,000 kDa is 1 µg/mL to 1 mg/mL.

14. The medium according to any one of claims 8 to 13, wherein the medium is a feeder-free medium.

15. The medium according to claim 14, wherein the medium is a serum-free medium.

16. A method for culturing a stem cell, comprising suspension culturing the stem cell in a medium for culturing stem cells comprising a polysaccharide other than dextran sulfate having a molecular weight of 4,000 kDa to 40,000 kDa.

17. The method according to claim 16, wherein the stem cell is one kind or two or more kinds selected from the group consisting of an adult stem cell, an embryonic stem cell and an induced pluripotent stem cell.

18. The method according to claim 16 or 17, wherein the polysaccharide other than the dextran sulfate having a molecular weight of 4,000 kDa to 40,000 kDa is the aforementioned polysaccharide other than the dextran sulfate, and is an anionic polysaccharide having a negatively-charged functional group or a salt thereof.

19. The method according to any one of claims 16 to 18, wherein the polysaccharide other than the dextran sulfate having a molecular weight of 4,000 kDa to 40,000 kDa is a sulfated polysaccharide other than the aforementioned dextran sulfate, or a salt thereof.

20. The method according to any one of claims 16 to 19, wherein the polysaccharide other than the dextran sulfate having a molecular weight of 4,000 kDa to 40,000 kDa is one kind or two or more kinds selected from the group consisting of heparin and a salt thereof, and dextran sulfate having a weight average molecular weight of 5,000 to 50,000 and a salt thereof.

21. The method according to any one of claims 16 to 20, wherein a content of the polysaccharide other than dextran sulfate having a molecular weight of 4,000 kDa to 40,000 kDa in the medium for culturing stem cells is 1 µg/mL to 1 mg/mL.

22. The method according to any one of claims 16 to 21, wherein the medium for culturing stem cells is a feeder free medium.

23. The method according to claim 22, wherein the medium for culturing stem cells is a serum-free medium.
